**Europäisches Patentamt**

**European Patent Office**

**Office européen des brevets**

(19)

(11) Veröffentlichungsnummer : **0 420 049 B1**

(12)

# EUROPÄISCHE PATENTSCHRIFT

(45) Veröffentlichungstag der Patentschrift :
**04.08.93 Patentblatt 93/31**

(51) Int. Cl.$^5$ : **A61K 37/66,** A61K 47/26,
A61K 47/28

(21) Anmeldenummer : **90118132.1**

(22) Anmeldetag : **20.09.90**

(54) **Stabilisierte Leukocyten-Interferone.**

(30) Priorität : **28.09.89 CH 3514/89**

(43) Veröffentlichungstag der Anmeldung :
**03.04.91 Patentblatt 91/14**

(45) Bekanntmachung des Hinweises auf die
Patenterteilung :
**04.08.93 Patentblatt 93/31**

(84) Benannte Vertragsstaaten :
**AT BE CH DE DK ES FR GB GR IT LI LU NL SE**

(56) Entgegenhaltungen :
**EP-A- 0 080 879**
**EP-A- 0 124 018**

(56) Entgegenhaltungen :
**EP-A- 0 133 767**
**EP-A- 0 196 203**
**FR-A- 2 510 409**
**US-A- 4 816 568**

(73) Patentinhaber : **F. HOFFMANN-LA ROCHE AG**
**Postfach 3255**
**CH-4002 Basel (CH)**

(72) Erfinder : **Ferro, Alberto, Dr.**
**Gstaltenrainweg 67**
**CH-4125 Riehen (CH)**

(74) Vertreter : **Lederer, Franz, Dr. et al**
**Patentanwalt Dr. Franz Lederer**
**Lucile-Grahn-Strasse 22**
**W-8000 München 80 (DE)**

EP 0 420 049 B1

**Beschreibung**

Die vorliegende Erfindung betrifft die Stabilisierung von Leukocyten-Interferon (IFN-α), insbesondere in Form von Lyophilisaten, mittels Disacchariden und Gallensäure bzw. einem Gallensäurederivat.

Unter IFN-α versteht man ein körpereigenes Protein mit antiviraler und immunregulatorischer Wirkung. Der antivirale Effekt wird dabei nicht durch eine direkte Beeinflussung der Viren selbst erzielt, sondern durch eine Wirkung auf deren Zielzellen im Sinne eines Schutzes gegen die Virusinfektion. Neben der antiviralen Aktivität kann α-Interferon objektivierbare Effekte auf Krebsgeschwülste ausüben, die es für den Einsatz in der Krebstherapie geeignet machen und es beeinflusst das körpereigene Immunsystem, indem es z.B. Makrophagen und NK-Zellen aktiviert und die Expression verschiedener immunologisch bedeutsamer Bestandteile der Zellmembran verstärkt.

IFN-α kann heute dank rekombinanter DNA-Technologie auf mikrobiellem Wege in Mengen hergestellt werden, die früher trotz grösster Anstrengungen durch Isolierung aus natürlichem Material (Leukocyten, Lymphocyten) und Reinigung nicht zur Verfügung gestellt werden konnten.

Erst diese neue Technologie hat daher einen Weg für die intensive klinische Erprobung und allfällige breite therapeutische Anwendung des IFN-α geöffnet und macht eine hinreichende Versorgung der für eine Behandlung in Frage kommenden Personen mit der Wirksubstanz möglich.

Es hat sich nun herausgestellt, dass IFN-α in reiner Form nicht völlig stabil ist und seine biologische Aktivität während der Lagerung Einbussen erleidet.

Zur Stabilisierung von IFN-α wurden daher bereits verschiedene Hilfsstoffe verwendet. Eine gewisse Stabilisierung von IFN-α wurde durch den Zusatz von Glycin erreicht (siehe z.B. publ. PCT-Anmeldung 86/00531 und europäisches Patent, Publ.-Nr. 82 481), wobei aber vorzugsweise gleichzeitig Albumin zugesetzt wird.

Im Bestreben, eine gute Stabilisierung von IFN-α über möglichst lange Zeit unter Vermeidung der Verwendung hochmolekularer Verbindungen wie Serumalbumin, Gelatine, Dextran oder Stärkederivate zu erreichen, wurde gefunden, dass dies mittels Disacchariden und Gallensäure bzw. einem Gallensäurederivat gelingt.

Die vorliegende Erfindung betrifft daher Zusammensetzungen, insbesondere in Form von Lyophilisaten, die ein Disaccharid und Gallensäure bzw. Gallensäurederivate enthalten, sowie Verfahren zu deren Herstellung, das darin besteht, dass man eine IFN-α-Lösung mit einem Disaccharid und einer Gallensäure oder einem Gallensäurederivat versetzt und die erhaltene Lösung gewünschtenfalls lyophilisiert. Die vorliegende Erfindung betrifft ausserdem pharmazeutische Präparate auf der Basis der erfindungsgemässen Zusammensetzungen sowie die Verwendung von Disacchariden und Gallensäure bzw. Gallensäurederivaten zur Stabilisierung von IFN-α.

Die erfindungsgemässen Zusammensetzungen können zur Herstellung eines pharmazeutischen Präparates zur Therapie bzw. Prophylaxe von viralen Infektionen und immunregulatorischer Anomalien, insbesondere Neoplasmen verwendet werden.

Die Stabilisierung kann auf natürliches oder rekombinantes IFN-α (r-IFN-α) angewendet werden. Ein besonders bevorzugtes IFN-α im Zusammenhang mit der vorliegenden Erfindung ist rekombinantes IFN-αA (r-IFN-αA). Der Gehalt der erfindungsgemässen Zusammensetzungen an IFN-α ist nicht kritisch. Der Konzentrationsbereich erstreckt sich über mehrere Zehnerpotenzen und wird nach oben im wesentlichen nur durch die Löslichkeit von IFN-α beschränkt. Für Human-IFN-α kommen beispielsweise Konzentrationen bis $5 \cdot 10^8$ internationale Einheiten (I.E.)/ml in Frage, wobei ein bevorzugter Bereich $0,1 \times 10^6$ bis $1 \times 10^8$ I.E./ml, insbesondere $1 \times 10^6$ bis $5 \times 10^7$ I.E./ml beträgt.

Das Disaccharid, vorzugsweise Lactose oder Saccharose, wird der IFN-α-Lösung in einer Konzentration von 0.5% bis 15% (Gew./vol.), vorzugsweise 5% (Gew./vol.), zugesetzt.

Die Gallensäure bzw. das Gallensäurederivat kann z.B. Cholsäure, Deoxycholsäure, Glycodeoxycholsäure, Taurodeoxycholsäure, Chenodeoxycholsäure, Glycochenodeoxycholsäure, Taurochenodeoxycholsäure, Glycocholsäure oder Taurocholsäure sein, wobei Glycocholsäure besonders bevorzugt ist. Es wird der IFN-α-Lösung in einer Konzentration von 0.01 bis 3% (Gew./vol.), vorzugsweise 0.5% (Gew./vol.) zugesetzt.

Weitere Hilfsstoffe zur pH-Einstellung, z.B. NaOH, pH-Pufferung, z.B. Phosphat- und Citrat-Puffer, Isotonisierung, z.B. Natriumchlorid und Konservierung des Präparates, z.B. Methyl- und Propyl-p-Hydroxybenzoesäureester, sowie zur verstärkung des Gerüstes des Lyophilisates, z.B. Glycin, können ebenfalls zugegeben werden.

In den folgenden Beispielen werden Einzelheiten der Erfindung beschrieben.

Das in den Beispielen verwendete r-IFN-αA kann in reiner Form nach bekannten, in der Literatur beschriebenen Verfahren oder nach für den Fachmann naheliegenden Verfahren erhalten werden.

Zur Bestimmung der antiviralen Aktivität des r-IFN-aA wurde ein cytopathologischer Test mit MDBK (Madison Darby Bovine Kidney)-Zellen und VSV (vescicular stomatitis virus)-Viren eingesetzt, der von Rubinstein et al. [J.Virol. 37, 755-758 (1981)] beschrieben worden ist. MDBK-Zellen und VSV-Viren sind allgemein zugänglich und können z.B. von der American Type Culture Collection (ATCC) bezogen werden (MDBK: ATCC Nrn. CCL 21 und CRL 6071; VSV: ATCC Nr. VR-1 59).

Für die Stabilitätsprüfung wurden Lösungen mit r-IFN-αA und den zu untersuchenden Zusätzen lyophilisiert und bei unterschiedlichen Temperaturen (5°, 25°, 35°, 45°, 55° und 65°C) aufbewahrt. Nach gewissen Zeitintervallen wurde die in den Lyophilisaten noch vorhandene antivirale Aktivität des IFN-αA bestimmt.

Die folgenden Beispiele illustrieren die Erfindung ohne sie einzuschränken.

Beispeil 1

3 Mio. I.E. r-IFN-αA wurden in je 1ml Wasser enthaltend Glycocholsäure, Lactose und weitere Hilfsmittel (siehe Tabelle 1) aufgenommen. Die erhaltenen Lösungen wurden sterilfiltriert (Membranfilter, 0,2 μm Porengrösse) und in 10 ml Glasfläschchen in einen dampfsterilisierten Gefriertrockner eingeladen. Nach Einfrieren der Lösungen bei -40°C während 4 h wurde die Primärtrocknung des Lyophilisationsprozesses bei ca. -30°C unter einem Druck von 0,1 mbar während 14 h durchgeführt. Anschliessend wurde die Sekundärtrocknung unter Vollvakuum bei +20°C während ca. 4 h durchgeführt. Die Glasfläschchen wurden im Gefriertrockner unter N$_2$-Atmosphäre mit geeigneten Aluminium-Kappen verschlossen. Sie wurden anschliessend verschiedenen Stabilitätstests unterworfen, deren Resultate in Tabelle 1 zusammengefasst sind.

## Tabelle 1

### Zusammensetzung der r-IFN-αA enthaltenden Lyophilisate

Lyophilisat aus:

| | | | | |
|---|---|---|---|---|
| r-IFN-αA (Mio.I.E.) | 1 | 3 | 9 | 18 |
| Glycocholsäure (mg) | 5.0 | 5.0 | 5.0 | 5.0 |
| Lactose (mg) | 50.0 | 50.0 | 50.0 | 50.0 |
| Natriumchlorid (mg) | 2.5 | 2.5 | 2.5 | 2.5 |
| NaOH 1 N ad pH 7.4 | q.s. | q.s. | q.s. | q.s. |
| Wasser zu Injektions- zwecken ad | 1.0 ml | 1.0 ml | 1.0 ml | 1.0 ml |

### Antivirale Aktivität (in Mio.I.E.) nach

| | | | | |
|---|---|---|---|---|
| Herstellung der Lyophilisate | 0.96 | 2.97 | 8.56 | 18.8 |
| 14 Tagen / 5°C | 0.85 | – | – | – |
| " / RT | 0.92 | – | – | – |
| " /35°C | 0.85 | – | – | – |
| " /45°C | 0.83 | – | – | – |
| 6 Monaten / 5°C | 1.02 | 2.89 | 9.2 | 18.7 |
| " / RT | 1.1 | 2.82 | 8.7 | 18.7 |
| " /35°C | 1.06 | 3.11 | 8.9 | 18.3 |
| " /45°C | 0.99 | 2.71 | 8.94 | n.d. |

**Patentansprüche**

**Patentansprüche für folgende Vertragsstaaten : AT, BE, CH, DE, DK, FR, GB, IT, LI, LU, NL, SE**

1. Zusammensetzung enthaltend $\alpha$-Interferon, ein Disaccharid und eine Gallensäure bzw. ein Gallensäurederivat.

2. Zusammensetzung nach Anspruch 1, dadurch gekennzeichnet, dass das $\alpha$-Interferon ein natürliches oder ein rekombinantes humanes Leukocyten-Interferon ist.

3. Zusammensetzung nach Anspruch 2, dadurch gekennzeichnet, dass sie r-IFN-$\alpha$A enthält.

4. Zusammensetzung gemäss einem der Ansprüche 1 bis 3, dadurch gekennzeichnet, dass es sich um ein Lyophilisat handelt.

5. Zusammensetzung gemäss einem der Ansprüche 1 bis 4, dadurch gekennzeichnet, dass das Disaccharid Lactose oder Saccharose ist.

6. Zusammensetzung gemäss einem der Ansprüche 1 bis 5, dadurch gekennzeichnet, dass sie 0.5% bis 15% (Gew./Vol.) Lactose oder Saccharose enthält.

7. Zusammensetzung gemäss einem der Ansprüche 1 bis 5, dadurch gekennzeichnet, dass sie 5% (Gew./Vol.) Lactose oder Saccharose enthält.

8. Zusammensetzung gemäss einem der Ansprüche 1 bis 6, dadurch gekennzeichnet, dass sie 0.01% bis 3.0% (Gew./Vol.) Gallensäure bzw. Gallensäurederivat, vorzugsweise 0.1% bis 1.0% (Gew.Vol.) Glycocholsäure enthält.

9. Zusammensetzung gemäss einem der Ansprüche 1 bis 6, dadurch gekennzeichnet, dass sie 0.5% (Gew./Vol.) Gallensäurederivat, vorzugsweise 0.5% (Gew./Vol.) Glycocholsäure, enthält.

10. Zusammensetzung enthaltend r-IFN-$\alpha$A, 5% (Gew./Vol.) Lactose und 0.5% (Gew./Vol.) Glycocholsäure.

11. Zusammensetzung gemäss einem der Ansprüche 1-10, als pharmazeutischer Wirkstoff.

12. Zusammensetzung gemäss einem der Ansprüche 1-10, als antiviraler und immunoregulatorischer Wirkstoff.

13. Verfahren zur Herstellung einer Zusammensetzung nach einem der Ansprüche 1 bis 10, dadurch gekenn-zeichnet, dass man $\alpha$-Interferon mit einem Disaccharid und einer Gallensäure bzw. einem Gallensäurederivat, vorzugsweise Glycocholsäure, versetzt und die erhaltene Lösung gewünschtenfalls lyophilisiert.

14. Pharmazeutische Präparate auf der Basis einer Zusammensetzung nach einem der Ansprüche 1 bis 10.

15. Verwendung von Disacchariden und Gallensäure bzw. Gallensäurederivaten, vorzugsweise Glycocholsäure, zur Stabilisierung von $\alpha$-Interferon.

16. Verwendung einer Zusammensetzung nach einem der Ansprüche 1-10 zur Herstellung eines pharmazeutischen Präparates zur Therapie bzw. Prophylaxe von Krankheiten.

17. Verwendung einer Zusammensetzung nach einem der Ansprüche 1-10 zur Herstellung eines pharmazeutischen Präparates zur Therapie bzw. Prophylaxe von viralen Infektionen.

18. Verwendung einer Zusammensetzung nach einem der Ansprüche 1-10 zur Herstellung eines pharmazeutischen Präparates zur Therapie bzw. Prophylaxe von immunregulatorischen Anomalien.

19. Verwendung einer Zusammensetzung nach einem der Ansprüche 1-10 zur Herstellung eines pharmazeutischen Präparates zur Therapie bzw. Prophylaxe von Neoplasmen.

**Patentansprüche für folgende Vertragsstaaten : ES, GR**

1. Verfahren zur Herstellung einer Zusammensetzung enthaltend α-Interferon, ein Disaccharid und eine Gallensäure bzw. ein Gallensäurederivat, dadurch gekennzeichnet, dass man α-Interferon mit einem Disaccharid und ggf. einer Gallensäure bzw. einem Gallensäurederivat, vorzugsweise Glycocholsäure, versetzt und die erhaltene Lösung gewünschtenfalls lyophilisiert.

2. Verfahren gemäss Anspruch 1, dadurch gekennzeichnet, dass als α-Interferon ein natürliches oder ein rekombinantes humanes Leukocyten-Interferon, vorzugsweise r-IFN-αA, verwendet wird.

3. Verfahren gemäss Anspruch 1 und 2, dadurch gekennzeichnet, dass als Disaccharid Lactose oder Saccharose mit einer Konzentration von 0.5% bis 15% (Gew./Vol.), vorzugsweise 5% (Gew./Vol.), verwendet wird.

4. Verfahren gemäss einem der Ansprüche 1-3, dadurch gekennzeichnet, dass eine Gallensäure bzw. ein Gallensäurederivat mit einer Konzentration von 0.01% bis 3.0% (Gew./Vol.), vorzugsweise 0.1% bis 1% (Gew./Vol) Glycocholsäure, verwendet wird.

5. Verfahren gemäss Anspruch 4, dadurch gekennzeichnet, dass ein Gallensäurederivat mit einer Konzentration von 0.5% (Gew./Vol.), vorzugsweise 0.5% (Gew./Vol) Glycocholsäure, verwendet wird.

6. Verfahren gemäss Anspruch 1, dadurch gekennzeichnet, dass r-IFN-αA, 5% (Gew./Vol.) Lactose und 0.5% (Gew./Vol.) Glycocholsäure verwendet wird.

7. Verwendung von Disacchariden, vorzugsweise Lactose oder Saccharose mit einer Konzentration von 0.5% bis 15% (Gew./Vol.), und ggf. Gallensäuren bzw. Gallensäurederivaten, vorzugsweise Glycocholsäure mit einer Konzentration von 0.1% bis 1% (Gew./Vol.), zur Stabilisierung von α-Interferon.

8. Verwendung einer Zusammensetzung enthaltend α-Interferon, ein Disaccharid, vorzugsweise Lactose oder Saccharose mit einer Konzentration von 0.5% bis 15% (Gew./Vol.), und eine Gallensäure bzw. ein Gallensäurederivat, vorzugsweise Glycocholsäure mit einer Konzentration von 0.1% bis 1% (Gew./Vol.), zur Herstellung eines pharmazeutischen Präparates zur Therapie bzw. Prophylaxe von Krankheiten.

9. Verwendung einer Zusammensetzung nach Anspruch 8 zur Herstellung eines pharmazeutischen Präparates zur Therapie bzw. Prophylaxe von viralen Infektionen.

10. Verwendung einer Zusammensetzung nach Anspruch 8 zur Herstellung eines pharmazeutischen Präparates zur Therapie bzw. Prophylaxe von immunregulatorischen Anomalien.

11. Verwendung einer Zusammensetzung nach Anspruch 8 zur Herstellung eines pharmazeutischen Präparates zur Therapie bzw. Prophylaxe von Neoplasmen.

12. Verwendung einer Zusammensetzung enthaltend r-IFN-αA, 5% (Gew./Vol.) Lactose und 0.5% (Gew./Vol.) Glycocholsäure nach einem der Ansprüche 8-11.


**Claims**

**Claims for the following Contracting States : AT, BE, CH, DE, DK, FR, GB, IT, LI, LU, NE, SE**

1. A composition containing α-interferon, a disaccharide and a bile acid or a bile acid derivative.

2. A composition according to claim 1, characterized in that the α-interferon is a natural or a recombinant human leucocyte interferon.

3. A composition according to claim 2, characterized in that it contains r-IFN-αA.

4. A composition according to any one of claims 1 to 3, characterized in that it is a lyophilizate.

5. A composition according to any one of claims 1 to 4, characterized in that the disaccharide is lactose or saccharose.

6. A composition according to any one of claims 1 to 5, characterized in that it contains 0.5% to 15% (wt./vol.) of lactose or saccharose.

7. A composition according to any one of claims 1 to 5, characterized in that it contains 5% (wt./vol.) of lactose or saccharose.

8. A composition according to any one of claims 1 to 6, characterized in that it contains 0.01% to 3.0% (wt./vol.) of bile acid or bile acid derivative, preferably 0.1% to 1.0% (wt. vol.) of glycocholic acid.

9. A composition according to any one of claims 1 to 6, characterized in that it contains 0.5% (wt./vol.) of bile acid derivative, preferably 0.5% (wt./vol.) of glycocholic acid.

10. A composition containing r-IFN-$\alpha$A, 5% (wt./vol.) of lactose and 0.5% (wt./vol.) of glycocholic acid.

11. A composition according to any one of claims 1-10 as a pharmaceutically active material.

12. A composition according to any one of claims 1-10 as an antivirally or immunoregulatorily active material.

13. A process for the manufacture of a composition according to any one of claims 1 to 10, characterized by treating $\alpha$-interferon with a disaccharide and a bile acid or a bile acid derivative, especially glycocholic acid, and, if desired, lyophilizing the solution obtained.

14. Pharmaceutical preparations based on a composition according to any on of claims 1 to 10.

15. The use of disaccharides and bile acids or bile acid derivatives, especially glycocholic acid, for the stabilization of $\alpha$-interferon.

16. The use of a composition according to any one of claims 1-10 for the manufacture of a pharmaceutical preparation for the therapy or prophylaxis of illnesses.

17. The use of a composition according to any one of claims 1-10 for the manufacture of a pharmaceutical preparation for the therapy or prophylaxis of viral infections.

18. The use of a composition according to any one of claims 1-10 for the manufacture of a pharmaceutical preparation for the therapy or prophylaxis of immunoregulatory anomalies.

19. The use of a composition according to any one of claims 1-10 for the manufacture of a pharmaceutical preparation for the therapy or prophylaxis of neoplasms.

**Claims for the following Contracting States : ES, GR**

1. A process for the manufacture of a composition containing $\alpha$-interferon, a disaccharide and a bile acid or a bile acid derivative, characterized by treating $\alpha$-interferon with a disaccharide and opt. a bile or a bile acid derivative, especially glycocholic acid, and, if desired, lyophilizing the solution obtained.

2. A process according to claim 1, characterized in that a natural or a recombinant human leucocyte interferon, preferably r-IFN-$\alpha$A, is used as the $\alpha$-interferon.

3. A process according to claim 1 and 2, characterized in that lactose or saccharose in a concentration of 0.5% to 15% (wt./vol.), preferably 5% (wt./vol.), is used as the disaccharide.

4. A process according to any one of claims 1-3, characterized in that a bile acid or a bile acid derivative in a concentration of 0.01% to 3.0% (wt./vol.), preferably 0.1% to 1.0% (wt. vol.) of glycocholic acid, is used.

5. A process according to claim 4, characterized in that a bile acid derivative in a concentration of 0.5% (wt./vol.), preferably 0.5% (wt./vol.) of glycocholic acid, is used.

6. A process according to claim 1, characterized in that r-IFN-$\alpha$A, 5% (wt./vol.) of lactose and 0.5% (wt./vol.) of glycocholic acid are used.

7. The use of disaccharides, preferably lactose or saccharose in a concentration of 0.5% to 15% (wt./vol.) and opt. bile acids or bile derivatives, preferably glycocholic acid in a concentration of 0.1% to 1% (wt./vol.), for the stabilization of α-interferon.

8. The use of a composition containing α-interferon, a disaccharide, preferably lactose or saccharose in a concentration of 0.5% to 15% (wt./vol.) and a bile acid or bile acid derivative, preferably glycocholic acid in a concentration of 0.1% to 1% (wt./vol.), for the manufacture of a pharmaceutical preparation for the therapy or prophylaxis of illnesses.

9. The use of a composition according to claim 8 for the manufacture of a pharmaceutical preparation for the therapy or prophylaxis of viral infections.

10. The use of a composition according to claim 8 for the manufacture of a pharmaceutical preparation for the therapy or prophylaxis of immunoregulatory anomalies.

11. The use of a composition according to claim 8 for the manufacture of a pharmaceutical preparation for the therapy or prophylaxis of neoplasms.

12. The use of a composition containing r-IFN-αA, 5% (wt./vol.) lactose and 0.5% (wt./vol.) glycocholic acid according to any one of claims 8-11.


**Revendications**

**Revendications pour les Etats contractants suivants : AT, BE, CH, DE, DK, FR, GB, IT, LI, LU, NL, SE**

1. Compositions contenant l'interféron α , un disaccharide et un acide biliaire ou un dérivé d'acide biliaire.

2. Composition selon la revendication 1, caractérisée en ce que l'interféron α est un interféron de leucocyte humain naturel ou recombinant.

3. Composition selon la revendication 2, caractérisée en ce qu'elle contient le r-IFN-αA.

4. Composition selon l'une des revendications 1 à 3, caractérisée en ce qu'il s'agit d'un produit de lyophilisation.

5. Composition selon l'une des revendications 1 à 4, caractérisée en ce que le disaccharide est le lactose ou le saccharose.

6. Composition selon l'une des revendications 1 à 5, caractérisée en ce qu'elle contient de 0.5% à 15% (poids/volume) de lactose ou de saccharose.

7. Composition selon l'une des revendications 1 à 5, caractérisée en ce qu'elle contient 5% (poids/volume) de lactose ou de saccharose.

8. Composition selon l'une des revendications 1 à 6, caractérisée en ce qu'elle contient de 0,01% à 3,0% (poids/volume) d'acide biliaire ou de dérivé d'acide biliaire de préférence de 0,1% à 1,0% (poids/volume) d'acide glycocholique.

9. Composition selon l'une des revendications 1 à 6, caractérisée en ce qu'elle contient 0,5% (poids/volume) de dérivé d'acide biliaire de préférence 0,5% (poids/volume) d'acide glycocholique.

10. Composition contenant le r-IFN-αA, 5% (poids/volume) de lactose et 0,5% (poids/volume) d'acide glycocholique.

11. Composition selon l'une des revendications 1-10 comme substance active pharmaceutique.

12. Composition selon l'une des revendications 1-10 comme substance active antivirale et immuno-régulatoire.

**13.** Procédé de préparation d'une composition selon l'une des revendications 1 à 10, caractérisé en ce qu'on mélange l'interféron α avec un disaccharide et un acide biliaire ou un dérivé d'acide biliaire de préférence l'acide glycocholique, et en ce qu'on lyophilise la solution obtenue si on le désire.

**14.** Préparation pharmaceutique à base d'une composition selon l'une des revendications 1 à 10.

**15.** Application de disaccharides et d'acide biliaire ou de dérivés d'acide biliaire, de préférence d'acide glycocholique, à la stabilisation de l'interféron α.

**16.** Application d'une composition selon l'une des revendications 1-10 à la préparation d'une préparation pharmaceutique pour le traitement ou la prophylaxie des maladies.

**17.** Application d'une composition selon l'une des revendications 1-10 à la préparation d'une préparation pharmaceutique pour le traitement ou la prophylaxie des infections virales.

**18.** Application d'une composition selon l'une des revendications 1-10 à la préparation d'une préparation pharmaceutique pour le traitement ou la prophylaxie des anomalies immunorégulatoires.

**19.** Application d'une composition selon l'une des revendication 1-10 à la préparation d'une préparation pharmaceutique pour le traitement ou la prophylaxie des néoplasies.

**Revendications pour les Etats contractants suivants : ES, GR**

**1.** Procédé de préparation d'une composition contenant l'interféron α, un disaccharide et un acide ou un dérivé d'acide biliaire caractérisé en ce qu'on mélange l'interféron α avec un disaccharide et le cas échéant un acide biliaire ou un dérivé d'acide biliaire de préférence l'acide glycocholique, et en ce que, si on le désire, on lyophilise la solution obtenue.

**2.** Procédé selon la revendication 1, caractérisé en ce qu'on utilise comme interféron α un interféron de leucocyte humain natural ou recombinant, de préférence le r-IFN-αA.

**3.** Procédé selon les revendications 1 et 2, caractérisé en ce qu'on utilise comme disaccharide le lactose ou le saccharose à une concentration de 0,5% à 15% (poids/volume), de préférence 5% (poids/volume).

**4.** Procédé selon l'une des revendications 1-3, caractérisé en ce qu'on utilise en acide biliaire ou un dérivé d'acide biliaire à une concentration de 0,01% à 3,0% (poids/volume), de préférence 0,1% à 1% (poids/volume) d'acide glycocholique.

**5.** Procédé selon la revendication 4, caractérisé en ce qu'on utilise un dérivé d'acide biliaire à une concentration de 0,5% (poids/vvolume), de préférence 0,5% (poids/volume) d'acide glycocholique.

**6.** Procédé selon la revendication 1, caractérisé en ce qu'on utilise le r-IFN-αA, 5% (poids/volume) de lactose et 0,5% (poids/volume) d'acide glycocholique.

**7.** Application de disaccharides, de préférence de lactose ou de saccharose à une concentration de 0,5% à 15% (poids/volume) et éventuellemnt d'acides biliaires ou de dérivés d'acide biliaire de préférence d'acide glycocholique à une concentration de 0,1% à 1% (poids/volume), à la stabilisation de l'interféron α.

**8.** Application d'une composition contenant l'interféron, α, un disaccharide, de préférence le lactose ou le saccharose à une concentration de 0,5% à 15% (poids/volume) et un acide biliaire ou un dérivé d'acide biliaire de préférence l'acide glycocholique à une concentration de 0,1% à 1% (poids/volume), à la préparation d'une préparation pharmaceutique pour le traitement ou la prophylaxie des maladies.

**9.** Application d'une composition selon la revendication 8 à la préparation d'une préparation pharmaceutique pour le traitement ou la prophylaxie des infections virales.

**10.** Application d'une composition selon la revendication 8 à la préparation d'une préparation pharmaceutique pour le traitement ou la prophylaxie des anomalies immunorégulatoires.

**11.** Application d'une composition selon la revendication 8 à la préparation d'une préparation pharmaceutique pour le traitement ou la prophylaxie des néoplasies.

12. Application d'une composition contenant le r-IFN-αA, 5% (poids/volume) de lactose et 0,5% (poids/volume) d'acide glycocholique selon l'une des revendications 8-11.